Europäisches Patentamt

European Patent Office

Office européen des brevets

(19)

(11) Publication number: 0 674 167 A1

# EUROPEAN PATENT APPLICATION

(21) Application number: 95103835.5

(22) Date of filing: 16.03.95

(51) Int. Cl.6: G01N 1/22

(30) Priority: 21.03.94 US 210963

(43) Date of publication of application:
27.09.95 Bulletin 95/39

(84) Designated Contracting States:
DE FR GB IT NL

(71) Applicant: TEXAS INSTRUMENTS
INCORPORATED
Mail Station 219,
13510 North Central Expressway
Dallas,
Texas 75243 (US)

(72) Inventor: Gorman, Lee V.
221 Aspen
Richardson,
Texas 75082 (US)
Inventor: Bachour, Kay
6711 Mossvine Circle
Dallas,
Texas 75240 (US)
Inventor: Cheng, Home-Been
2213 Primrose
Richardson,
Texas 75082 (US)

(74) Representative: Holt, Michael et al
Texas Instruments Limited,
Wellington House,
61-73 Staines Road West
Sunbury on Thames TW16 7AH (GB)

(54) Super cooled air emissions sampler for entrained contaminant capture.

(57) An air sample is taken into a heat exchanger where it is super-cooled so that the entrained contaminants are forced to change to a solid and coat out onto the internal surface of the heat exchanger while cleaned super-cooled air is disposed to the atmosphere. Thereafter, the solidified or liquefied sample is collected from the exchanger and forwarded to a laboratory for the needed or required analyses of each component. This event provides a sample of substance mass for analyses instead of an air sample in a balloon type container. Along with this, and concurrent with the sample solidification, an air velocity monitor, dew point detector and a computer to handle the calculations determine the air flow, the air density and the moisture content of the air mass that is represented by the sample.

FIG. 1

## BACKGROUND OF THE INVENTION

### FIELD OF THE INVENTION

This invention relates to a method and system for sampling and analyzing exhaust gases and, more specifically, to such a method wherein contaminants are solidified by a cooling event or are cryogenically removed from the ambient air or from an exhaust air stream over a known time period for analysis.

### BRIEF DESCRIPTION OF THE PRIOR ART

Clean air and environmental pollution control required by various laws that emissions at or in the places where chemical and material contaminants which can and do become entrained in the air, such as in exhausted air from equipment that produces or uses such substances, be assessed for assurance that the air is safe to breath and for assurance that the environment is protectod. Many types of apparatus are available to perform such emissions assessment, however all have some critical shortfall in producing an assessment that provides all of the following:

1. A representative sample of the contaminated air.
2. Analytical accuracy that is at the leading edge of technology.
3. Sampling diversity for multiple contaminants.
4. Capture of exact same time samples at several locations.
5. Automatic time averaging of sampled air quality.
6. Allows each contaminant to be assessed individually.

Some typical air contaminants are compounds such as acids, caustics, oils, organic solvents, refrigerants and various other substances such as resins, dust and liquid particulate pesticides, herbicides, fuels, chemical vapors and water-based materials.

The art of air steam, confined volumes and open air space analysis has progressed only slightly in the past decade. The U.S. Environmental Protection Agency (USEPA) as well as other regulatory entities, state and private institutions have promoted, approved and defined a number of air sampling methods that range from direct in situ analysis and plumbed sampling conduits from the air source to a central analytical facility to gather samplings in an air bag for transport to a remote laboratory for analysis. Some of these sampling approaches operate well where the air contains a single or only a few similar types of compounds. The major problem is that an air sample is difficult to assess in the analytical systems of the prior art. An air sample must in some way be adsorbed in a liquid or solidified in some way to attain accurate analytical results and to be able to realistically relate the laboratory or instrument data to a reference for beneficial units of measure, such as grams per second, pounds per hour, milligrams per cubic meter of air flow, volume or air space, etc. Actually, the art is weak when it comes to all of the issues stated above. No one method presently known will meet the accuracy and contaminant speciation required in present USEPA and state administrative codes which are the basis for air contaminant emission levels permitted and the control thereof. In addition, present air sampling techniques are excessively expensive as well as technically inconclusive.

### SUMMARY OF THE INVENTION

Briefly, in accordance with the present invention, there is provided a system which overcomes the above described disadvantages of the prior art by continuously taking an air sample into a heat exchanger wherein the contaminated air sample is super-cooled to below the freezing temperature of the lowest boiling contaminant. The super-cooling is to be at a sufficiently low enough temperature to cause the entrained contaminants in the air sample to change to the solid phase and coat out onto the internal surface of the heat exchanger while the cleaned, essentially contaminant-free, super-cooled air from the formerly contaminated air sample is expelled to the atmosphere. Thereafter, the solidified or liquefied contaminant sample is collected from the heat exchanger and forwarded to a laboratory for the required analysis of each component. This event provides a sample of substance mass for analysis instead of an air sample in a balloon type container. Concurrent with the contaminant sample collection and solidification event, an air velocity monitor, dew point detector and instrumentation for any other parameters in the air stream which it is desired to measure along with a computer to handle calculations determine the air flow, air density, the moisture content of the air mass which is represented by the sample being analyzed and any other parameters which it is desired to calculate from the data obtained and sent to the computer. This allows the laboratory results to be converted into usable terms such as, for example, parts per million by weight of the air mass from which the sample was taken and the rate of emissions in weight or volume per unit of time or other reference quantity. This is unique in sampling technology because it is a total representation of the air mass containing the contaminants while providing a true time-based analytical picture of the make-up of the air mass components and their mean average concentrations over the time period when the sample was taken. Under these conditions, the analytical data represents the mean average concentration of the air component contaminants for an extended and known time period.

BRIEF DESCRIPTION OF THE DRAWINGS

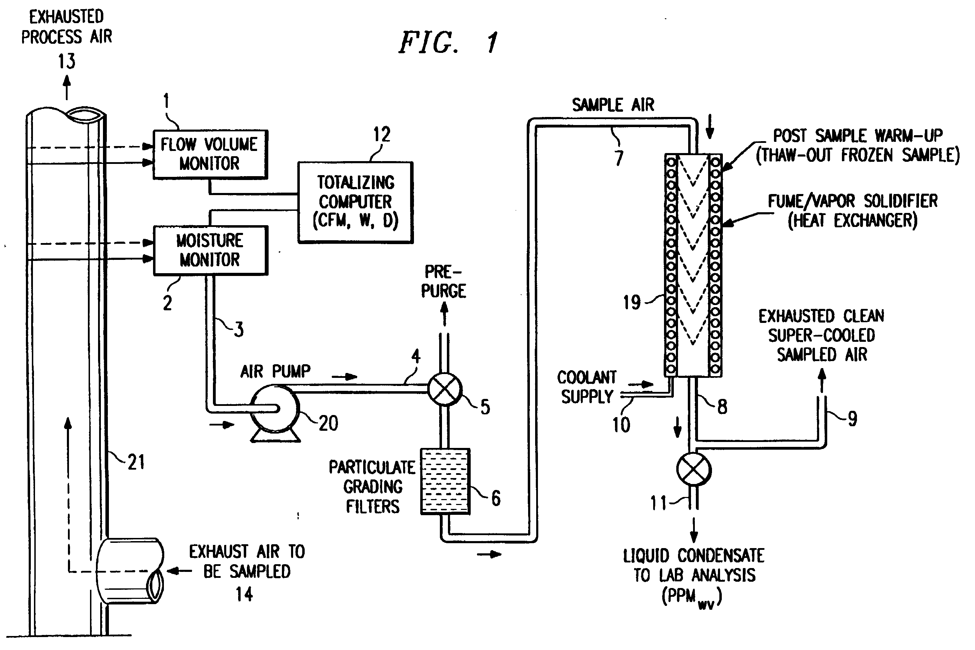

The FIGURE is a schematic diagram of a typical arrangement for collecting a sample of contaminants from an air stream being exhausted up and out of an exhaust stack in accordance with the present invention.

DESCRIPTION OF THE PREFERRED EMBODIMENT

Referring now to the FIGURE, there is shown a schematic diagram of a typical arrangement for collecting a sample of contaminants from an air stream being exhausted up and out of an exhaust stack in accordance with the present invention.

Exhaust air 14 which is to be expelled to the atmosphere and is to be sampled enters an exhaust stack 21, this air being monitored for desired parameters, such as, for example, moisture by a moisture monitor 2 and as to volume flow per unit of time (e.g. cubic feet per minute) by a volume flow monitor 1 before being exhausted to the atmosphere at stack outlet 13. This monitored information is transmitted to a computer 12 for data storage, totalization, averaging and any other desired operation available from the accumulated data including averaging over a sample collection time period. The air from the exhaust stack 21 is also measured for dew point and dry-bulb temperature, this information also being transmitted to the computer 12 for data storage and referencing to the air/water-vapor algorithm (such as the common psychrometric chart or table for the temperaturure range involved) to acquire the mean average moisture content and density of the exhausted air during the sample collection time period.

Air from the exhaust stack 21 in known quantity, as, for example, a known percentage of the total air being exhausted, is also drawn from the exhaust stack into the air pump 20 along the line 3 which communicates with the exhaust stack and is sent along the line 4 to a purge valve 5. The purge valve 5 is initially open, causing air in the line 4 to by-pass the contaminant collection system until the system is brought into stabilization and completes the pre-cooling cycle and before commencement of parameter measurement. When the system has been stabilized, parameter measurement and transmission to the computer 12 commences and the purge valve 5 is closed, causing all air in the line 4 to travel to the heat exchanger 19 after passing through a particulate grading filter 6 wherein particulate materials are removed, also for possible later sampling.

The contaminated air sample in lines 3,4 and 7 is still at or near the exhaust stack temperature and may be separately sampled from any one or more of these lines for particulate, organic and inorganic gases that do not condense and solidity readily due to technical preferences and/or cryogenic cooling capacity limitations of the heat exchanger 19. The air from the filters 6 passes along line 7 to the heat exchanger 19 where the air is super-cooled to a sub-freezing temperature to cause the entrained water vapor and preferably all of the condensable contaminants to solidity on the internal surfaces of the heat exchanger. Depending upon the degree of accuracy required, the temperature within the heat exchanger 19 can be lowered to near the temperature of liquid nitrogen to essentially extract by super-cooling all entrained vapors from the air stream and have dry or contaminant-free air exhausting from the heat exchanger along line 8 to the atmosphere. Any contaminants that do not solidity but do liquefy at the reduced temperatures provided in the heat exchanger 19 will travel to the outlet 11 from the heat exchanger and be collected for later analysis. Liquid nitrogen or other coolant is supplied for cooling the heat exchanger 19 either by conduction, radiation and/or convention, as best applies, to accomplish sample air contaminant super-cooling extraction and solidification. A coolant supply is shown being fed to the heat exchanger 19 along the coolant supply line 10 in the preferred embodiment.

After the sample extraction cycle is complete, the collected water and contaminant mass are withdrawn from the heat exchanger 19 for delivery to a laboratory for analysis. The sample mass may be forced into a sample container as a solid or liquefied in situ and allowed to gravity drain into a sample container either with or without an assisting plunger to promote sample removal from the heat exchanger.

The computer data collected in computer 12 during the sampling period is saved on a disk or other appropriate storage medium and forwarded along with the sample to the laboratory for the data reduction and air emissions calculations. The analytical data obtained from the sample being analyzed along with the data collected by the computer 12 are then converted into the appropriate data, such as, for example, pounds per hour to represent the actual air stream exhausting from the emissions source, such as a chemical process using acids and thus generating acidic vapors.

In summary, the system in accordance with the present invention captures an absolute duplicate representation of entrained chemical, compound or material contaminants from an air stream by means of rapid super-cooling to form a solid that may be delivered to a laboratory for state-of-the-art analysis at an accuracy which is quality controlled and certifiable to the lower detection limits as opposed to sample extraction and in situ analyses at the exhaust stack or field location. The sample is collected over an extended time period so that any momentary aberrations from the average in contaminant presence in the air stream are averaged out and will not result in erroneous readings as are possible when individual samples are taken over very short time periods, as in the prior art.

EXAMPLE

Typically, a 50 milliliter quantity or about 50 grams of sample are required for the analytical determinations. Since the amount of moisture in the air plays an important role in the sample collection volume, the heat exchanger and coolant flow may require adjustment accordingly. For example, Table 1 shows such changes in air flow to the heat exchanger unit 19 for collecting the required sample size based upon differences in sampling time as well as the difference between an air stream having 40% relative humidity and a water saturated air stream.

TABLE 1

| SAMPLING TIME HOURS | SATURATED AIR STREAM | | 40% R.H. AIR STREAM | |
|---|---|---|---|---|
| | CFM | CCM | CFM | CCM |
| 1 | 1.33 | 40,000 | 4.33 | 120,000 |
| 4 | 0.33 | 10,000 | 1.07 | 30,000 |
| 8 | 0.17 | 5,000 | 0.54 | 15,000 |
| 12 | 0.11 | 3,600 | 0.36 | 10,000 |
| 16 | 0.08 | 2,500 | 0.27 | 7,500 |
| 24 | 0.06 | 1,800 | 0.20 | 5,000 |

where CFM = cubic feet of air per minute and CCM = cubic centimeters of air per minute flow, both through the super-cooling heat exchanger 19.

Some of the key parameters, all or some of which would normally be calculated alter the analysis of the sample from the heat exchanger would be PPMwv (pounds of contaminant per million pounds of condensed vapor), PPMwa (pounds of contaminant per million pounds of exhausted dry air), D (specific total sample time mean average air density as pounds per cubic feet of bone dry air exhausted, CFM (total sample time mean average quantity of air exhausted in cubic feet per minute), ROE (rate of contaminant emitted into the air as pounds per minute). Then PPMwa = (PPMwv)(W) and ROE = (PPMwa)(CFM)(D). Therefore, if PPMwv was 10 and W = 0.625, and D = 0.0742 and CFM = 10,000, then PPMwa would be 6.25 and the ROE would be (6.25)(10,000)(0.0742)/1,000,000 = 0.00464 pounds/minute.

In accordance with the present invention, the expression of a quantity of an air emittant maybe determined on any basis with an accuracy not now attainable without difficulty to solve the data certification issues via laboratory analysis under controlled and calibrated expertise.

Though the invention has been described with respect to specific preferred embodiments thereof, many variations and modifications will immediately become apparent to those skilled in the art. It is therefore the intention that the appended claims be interpreted as broadly as possible in view of the prior art to include all such variations and modifications.

**Claims**

1. A method of analyzing a gas contaminated exhaust air stream comprising the steps of:
   (a) measuring predetermined properties of the gas contaminated exhaust air in said gas contaminated exhaust air stream;
   (b) extracting a minor portion of said gas contaminated exhaust air from said gas contaminated exhaust air stream;
   (c) cooling said extracted gas contaminated exhaust air to a temperature sufficiently low to condense said gas and provide a contaminant condensate; and
   (d) analyzing said contaminant condensate.

2. The method of claim 1, further including the step of determining a desired contaminant parameter in response to the analysis of said contaminant condensate in step (d) and said predetermined properties measured in step (a).

3. The method of claim 1 or claim 2 wherein said condensate is a liquid.

4. The method of any preceding claim, further including the steps of removing particulate contaminants from said extracted gas contaminated exhaust air and analyzing said particulate contaminants.

5. The method of any preceding claim wherein said extraction step (b) is a known amount of said gas contaminated exhaust air and wherein said extraction takes place over a known time period.

6. Apparatus for performing a method as claimed in any preceding claim.

7. A system for analyzing a gas contaminated exhaust air stream comprising:
   (a) instrumentation for measuring predetermined properties of the gas contaminated exhaust air in said gas contaminated exhaust air stream;
   (b) a gas extractor for extracting a portion of said gas contaminated exhaust air from said gas contaminated exhaust air stream;
   (c) a cooler for cooling said extracted gas contaminated exhaust air to a temperature sufficiently low to condense said gas and provide a contaminant condensate; and
   (d) analyzing means for analyzing said contaminant condensate.

8. The system of claim 7, further including calculating means for determining a desired contaminant parameter in response to the analysis of said contaminant condensate in (d) and said predetermined properties measured in (a).

9. The system of claim 7 or claim 8, further including a filter for removing particulate contaminants from said extracted gas contaminated exhaust air and means to analyze said particulate contaminants.

*FIG. 1*

EXHAUSTED PROCESS AIR
13

1 — FLOW VOLUME MONITOR

12 — TOTALIZING COMPUTER (CFM, W, D)

2 — MOISTURE MONITOR

3

AIR PUMP

20

4

PRE-PURGE

5

PARTICULATE GRADING FILTERS

6

21

EXHAUST AIR TO BE SAMPLED
14

SAMPLE AIR

7

POST SAMPLE WARM-UP (THAW-OUT FROZEN SAMPLE)

FUME/VAPOR SOLIDIFIER (HEAT EXCHANGER)

19

EXHAUSTED CLEAN SUPER-COOLED SAMPLED AIR

COOLANT SUPPLY

10

8

9

11

LIQUID CONDENSATE TO LAB ANALYSIS (PPM$_{wv}$)

EP 0 674 167 A1

European Patent
Office

**EUROPEAN SEARCH REPORT**

Application Number

EP 95 10 3835

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | DE-A-42 05 793 (GFA GESELLSCHAFT FÜR ARBEITSPLATZ- UND UMWELTANALYTIK MBH) * abstract; claim 1 * | 1,3-9 | G01N1/22 |
| Y | EP-A-0 470 345 (BK LADENBURG GMBH) * the whole document * | 1,3-9 | |
| A | DE-A-41 31 088 (SIEMENS AG) * abstract * | 1,7 | |
| A | US-A-4 386 534 (M. S. ENGLUND ET AL.) * abstract * * column 4, line 19 - line 22 * | 7 | |
| A | US-A-3 999 425 (L. T. COLLIN) * abstract * * column 4, line 38 * | 1,5 | |
| | | | **TECHNICAL FIELDS SEARCHED (Int.Cl.6)** |
| | | | G01N G01M |

The present search report has been drawn up for all claims

| Place of search | Date of completion of the search | Examiner |
|---|---|---|
| BERLIN | 4 July 1995 | Brison, O |

EPO FORM 1503 03.82 (P04C01)